(19) Europäisches Patentamt
European Patent Office
Office européen des brevets

(11) EP 4 339 591 A1

(12) EUROPEAN PATENT APPLICATION
published in accordance with Art. 153(4) EPC

(43) Date of publication:
20.03.2024 Bulletin 2024/12

(51) International Patent Classification (IPC):
G01N 15/14 (2024.01)    G01N 21/49 (2006.01)

(21) Application number: 22807299.7

(52) Cooperative Patent Classification (CPC):
G01N 15/14; G01N 21/49

(22) Date of filing: 14.04.2022

(86) International application number:
PCT/JP2022/017763

(87) International publication number:
WO 2022/239596 (17.11.2022 Gazette 2022/46)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 11.05.2021 JP 2021080332

(71) Applicant: ThinkCyte, Inc.
Bunkyo-ku, Tokyo 113-0033 (JP)

(72) Inventors:
• NAKAGAWA Keiji
  Tokyo 113-0033 (JP)
• TODA Keisuke
  Tokyo 113-0033 (JP)

(74) Representative: Ernest Gutmann - Yves
Plasseraud S.A.S.
c/o Plasseraud IP
66, rue de la Chaussée d'Antin
75440 Paris Cedex 09 (FR)

(54) FEATURE AMOUNT CALCULATION DEVICE, FEATURE AMOUNT CALCULATION METHOD, AND PROGRAM

(57) A feature value calculation device is a feature value calculation device that calculates a feature value indicating optical characteristics of an object to be measured in a flow cytometry method, the device including: a signal information acquisition unit to acquire signal information indicating a temporal change in intensity of the electromagnetic waves which is generated by a light detector receiving the modulated electromagnetic waves from the object to be measured which is present in a light irradiation region irradiated with the illumination light from the light source, by using any one or more of a configuration for structured illumination in which illumination light from a light source is structured as structured illumination light and radiated onto the object to be measured or a configuration for structured detection in which modulated electromagnetic waves from the object to be measured are structured and detected; a region setting unit that sets a region to be processed in waveform data in which a feature value is calculated in a domain of the waveform data based on the signal information acquired by the signal information acquisition unit; and a feature value calculation unit that calculates the feature value from the waveform data in the region set by the region setting unit.

FIG. 3

**Description**

[Technical Field]

The present invention relates to a feature value calculation device, a feature value calculation method, and a program.

Priority is claimed on Japanese Patent Application No. 2021-080332, filed May 11, 2021, the content of which is incorporated herein by reference.

[Background Art]

Flow cytometry is a widely used cell biology technique for using a laser to count, discriminate, and characterize cells in heterogeneous mixtures.

In a flow cytometer, objects to be measured such as cells pass through a laser light irradiation position in a line in a flowing liquid, and scattered light and fluorescence generated by irradiating the objects to be measured with irradiation light are detected by a detection device.

In conventional flow cytometers, a light detector was sometimes installed in front of illumination light with which an object to be measured was irradiated, and the intensity of light scattered in a forward direction (forward scattered light) was measured as the total amount (forward scatter: FSC). In addition, a light detector was sometimes installed on the side of illumination light with which an object to be measured was irradiated, and the intensity of light scattered in a side direction (side scattered light) was measured as the total amount (side scatter: SSC).

Since larger particles typically generate more scattered light in the forward direction than smaller particles, FSC has been used as an index for providing information about the size of particles. In addition, SSC is light that propagates in a direction different from the original optical path of illumination light with which an object to be measured is irradiated, and has been used as an index for providing information about the internal structure of cells such as granularity and complexity.

Ghost Cytometry (GC) technology is known as a technique that makes it possible to acquire richer and more detailed morphological information of cells than conventional flow cytometry (Patent Documents 1 and 2).

[Citation List]

[Patent Literature]

    [Patent Document 1]
    International Patent Publication WO 2016/136801
    [Patent Document 2]
    International Patent Publication WO 2017/073737

[Summary of Invention]

[Technical Problem]

In GC technology, as an example, an optical signal emitted by an object to be measured such as a cell when the object to be measured is irradiated with structured illumination light is used to acquire morphological information of the object to be measured. The optical signal acquired by a configuration for structured illumination includes compressed morphological information of the object to be measured which is irradiated with the structured illumination light. In a flow cytometer using GC technology, machine learning is performed using waveform data indicating a temporal change in the detected optical signal intensity as learning data as it is, and the object to be measured is predicted from the waveform data of the object to be measured on the basis of a created classification model, which makes it possible to perform faster and more accurate discrimination processing on the object to be measured. However, information relating to various optical characteristics other than the morphological information of the object to be measured included in an optical signal acquired by the configuration for structured illumination has not been acquired and used as a feature value until now. In GC technology, for example, there is a need to simultaneously acquire information relating to various optical characteristics as feature values in addition to the morphological information of the object to be measured from the optical signal emitted from the object to be measured when the object to be measured is irradiated with the structured illumination light.

The present invention was contrived in view of the above points, and provides a feature value calculation device, a feature value acquisition method, and a program that make it possible to calculate various feature values included in an optical signal detected in GC technology.

[Solution to Problem]

The present invention was contrived in order to solve the above-described problems. According to an aspect of the present invention, there is provided a feature value calculation device that calculates a feature value indicating optical characteristics of an object to be measured in a flow cytometry method, the device including: a signal information acquisition unit to acquire signal information indicating a temporal change in intensity of the electromagnetic waves which is generated by a light detector receiving the modulated electromagnetic waves from the object to be measured which is present in a light irradiation region irradiated with the illumination light from the light source, by using any one or more of a configuration for structured illumination in which illumination light from a light source is structured as structured illumination light and radiated onto the object to be measured or a configuration for structured detection in which modulated electromagnetic waves from the object to be measured are structured and detected; a region setting unit that sets a region to be processed in a waveform data in which a feature value is calculated in a domain of the waveform data based on the signal information acquired by the signal information acquisition unit; and a feature value calculation unit that calculates the feature value from the waveform data in the region set by the region setting unit.

In addition, according to an aspect of the present invention, in the above feature value calculation device, the waveform data is data indicating the intensity of the electromagnetic waves with elapsed time as a domain, and the region setting unit sets a region in a range of a specific elapsed time as the region in the waveform data.

In addition, according to an aspect of the present invention, in the above feature value calculation device, the waveform data is data indicating a signal intensity in a frequency spectrum with frequency as a domain, and the region setting unit sets a region in a range of a specific frequency as the region in the waveform data.

In addition, according to an aspect of the present invention, in the above feature value calculation device, measurement for performing discrimination based on morphological information of the object to be measured from the waveform data and measurement for calculating the feature value are executed in parallel on the basis of a discrimination model created in advance by learning a relationship between the waveform data and the morphological information of the object to be measured on the basis of machine learning.

In addition, according to an aspect of the present invention, in the above feature value calculation device, the signal information acquisition unit acquires, as the signal information, signal information indicating a temporal change in intensity of scattered light from the object to be measured which is present in the light irradiation region.

In addition, according to an aspect of the present invention, in the above feature value calculation device, the signal information acquisition unit acquires, as the signal information, signal information indicating a temporal change in intensity of scattered light from the object to be measured which is present in the light irradiation region, the waveform data is data indicating the intensity of the scattered light with elapsed time as a domain, the region setting unit sets a region in a range of a specific elapsed time as the region in the waveform data, and the feature value calculation unit calculates, as the feature value, an integrated value of the intensity of the scattered light received by the light detector in the range of the specific elapsed time from the waveform data in the region set by the region setting unit.

In addition, according to an aspect of the present invention, the above feature value calculation device further includes a conversion unit that converts the feature value into a value corresponding to intensity of electromagnetic waves acquired by a flow cytometer other than its own device on the basis of the feature value calculated by the feature value calculation unit and a measured value obtained by measurement of a reference object to be measured.

In addition, according to an aspect of the present invention, there is provided a method of calculating a feature value indicating optical characteristics of an object to be measured in a flow cytometry method, the method including: a signal information acquisition step of acquiring signal information indicating a temporal change in intensity of the electromagnetic waves which is generated by a light detector receiving the modulated electromagnetic waves from the object to be measured which is present in a light irradiation region irradiated with the illumination light from the light source, by using any one or more of a configuration for structured illumination in which illumination light from a light source is structured as structured illumination light and radiated onto the object to be measured or a configuration for structured detection in which modulated electromagnetic waves from the object to be measured are structured and detected; a region setting step of setting a region to be processed in waveform data in which a feature value is calculated in a domain of the waveform data based on the signal information acquired in the signal information acquisition step; and a feature value calculation step of calculating the feature value from the waveform data in the region set in the region setting step.

In addition, according to an aspect of the present invention, there is provided a program for causing a computer of a feature value calculation device that calculates a feature value indicating optical characteristics of an object to be measured in a flow cytometry method to execute: a signal information acquisition step of acquiring signal information indicating a temporal change in intensity of the electromagnetic waves which is generated by a light detector receiving the modulated electromagnetic waves from the object to be measured which is present in a light irradiation region irradiated with the illumination light from the light source, by using any one or more of a configuration for structured illumination in which illumination light from a light source is structured as structured illumination light and radiated onto the object to be measured or a configuration for structured detection in which modulated electromagnetic waves from the object to be measured are structured and detected; a region setting step of setting a region to be processed in waveform data in which a feature value is calculated in a domain of the waveform data based on the signal information acquired in the signal information acquisition step; and a feature value calculation step of calculating the feature value from the waveform data in the region set in the region setting step.

[Advantageous Effects of Invention]

According to the present invention, it is possible to acquire various types of information included in an optical signal detected in GC technology.

[Brief Description of Drawings]

FIG. 1 is a diagram illustrating an example of a flow cytometer according to a first embodiment of the present invention.
FIG. 2 is a diagram illustrating an example of a spatial light modulation unit according to the first embodiment of the present invention.
FIG. 3 is a diagram illustrating an example of a configuration of a feature value calculation device according to the first embodiment of the present invention.
FIG. 4 is a diagram illustrating an example of waveform data based on signal information in a case where the elapsed time is defined as a domain according to the first embodiment of the present invention.
FIG. 5 is a diagram illustrating an example of waveform data indicated by signal information in a case where the frequency is defined as a domain according to the first embodiment of the present invention.
FIG. 6 is a diagram illustrating an example of a feature value calculation process according to the first embodiment of the present invention.
FIG. 7 is a diagram illustrating a correlation between forward scattered light intensity and backward scattered light intensity detected by a flow cytometer according to Example 1 of the first embodiment of the present invention.
FIG. 8 is a diagram illustrating a correlation between the integrated value of a forward detected scattered light signal and the integrated value of a backward detected scattered light signal detected by the flow cytometer according to Example 1 of the first embodiment of the present invention.
FIG. 9 is a diagram illustrating an example of a configuration of a feature value calculation device according to a second embodiment of the present invention.
FIG. 10 is a diagram illustrating an example of a feature value calculation process according to the second embodiment of the present invention.
FIG. 11 is a diagram illustrating a correlation between the measured value of forward scattered light intensity and the integrated value of a forward detected scattered light signal according to Example 2 of the second embodiment of the present invention.
FIG. 12 is a diagram illustrating a correlation between the measured value of forward scattered light intensity and the integrated value of a converted forward detected scattered light signal according to Example 2 of the second embodiment of the present invention.

[Description of Embodiments]

(First embodiment)

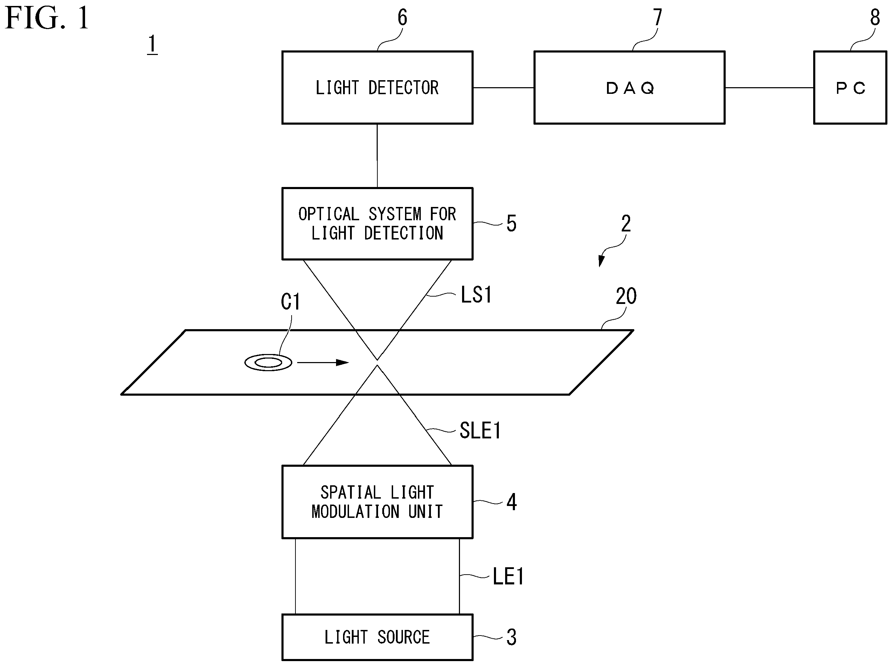

Hereinafter, an embodiment of the present invention will be described in detail with reference to the accompanying drawings. FIG. 1 is a diagram illustrating an example of a flow cytometer 1 according to the present embodiment. The flow cytometer is a measurement device that includes at least a microfluidic device provided with a flow path through which an object to be measured can flow together with a fluid, a light source that irradiates the flow path with illumination light, and a light detector that detects signal light emitted from the object to be measured flowing through the flow path by irradiating the object to be measured with the illumination light, and is configured such that the object to be measured flowing through the flow path together with a fluid is optically measured while moving in the flow path. The flow cytometer 1 according to the present embodiment includes a microfluidic device 2, a light source 3, a spatial light modulation unit 4, an optical system for light detection 5, a light detector 6, a data acquisition (DAQ) device 7, and a personal computer (PC) 8.

The microfluidic device 2 is provided with a flow path 20 through which an object to be measured C1 can flow together with a fluid. The object to be measured C1 is, for example, a cell. Meanwhile, the object to be measured C1 is not limited to cells insofar as it can flow through the flow path 20 together with a fluid. Other examples of the object to be measured C1 may include microparticles, spheroids (cell clusters), viruses, microorganisms such as microbacteria, cell counting beads for flow cytometry, simulated cells, and the like. The microparticles include pollen, microplastics, polymer particles, foreign substances contained in the object to be measured, and the like. The flow velocity of a fluid flowing through the flow path 20 is a constant flow velocity regardless of the type and individual differences of the object to be measured C1 flowing therethrough. In addition, although the microfluidic device 2 sequentially causes a plurality of objects to be measured to flow through the flow path 20, it is preferable that the number of objects to be measured that pass through the illumination light irradiation position in the flow path 20 at one time be one.

The microfluidic device 2 may have a sorting unit (not shown) for separating the object to be measured C1 further installed in the flow path 20 through which the object to be measured C1 can flow together with a fluid. In that case, the microfluidic device 2 discriminates the object to be measured C1 moving in the flow path on the basis of the result of optical measurement, and causes the sorting unit to sort the desired object to be measured C1 on the basis of the discrimination result.

The light source 3 and the spatial light modulation unit 4 function to radiate structured illumination light. The light source 3 and the spatial light modulation unit 4 irradiate structured illumination light SLE1 toward the object to be measured C1 that passes through the flow path 20 as will be described below.

Illumination light LE1 emitted from the light source 3 is converted into the structured illumination light SLE1 structured through the spatial light modulation unit 4, and is irradiated to the irradiation position in the flow path 20. The light source 3 is, for example, a laser light source, a semiconductor laser light source, or a light emitting diode (LED) light source. The illumination light LE1 emitted by the light source 3 may be spatially incoherent light, but spatially coherent light is preferable. In the present embodiment, the illumination light LE1 emitted by the light source 3 is, for example, spatially coherent light.

The spatial light modulation unit 4 is disposed on an optical path between the light source 3 and the light detector 6. In the present embodiment, the spatial light modulation unit 4 is disposed on an optical path between the light source 3 and the flow path 20. The configuration of this arrangement is also referred to as a configuration for structured illumination. The illumination light LE1 irradiated from the light source 3 is structured by the spatial light modulation unit 4, and the structured illumination light SLE1 is radiated to the flow path 20. In the configuration for structured illumination, the structured illumination light SLE1 is irradiated toward the object to be measured C1 so that a structured illumination pattern is imaged at the irradiation position in the flow path 20. Meanwhile, the structured illumination pattern will be described later.

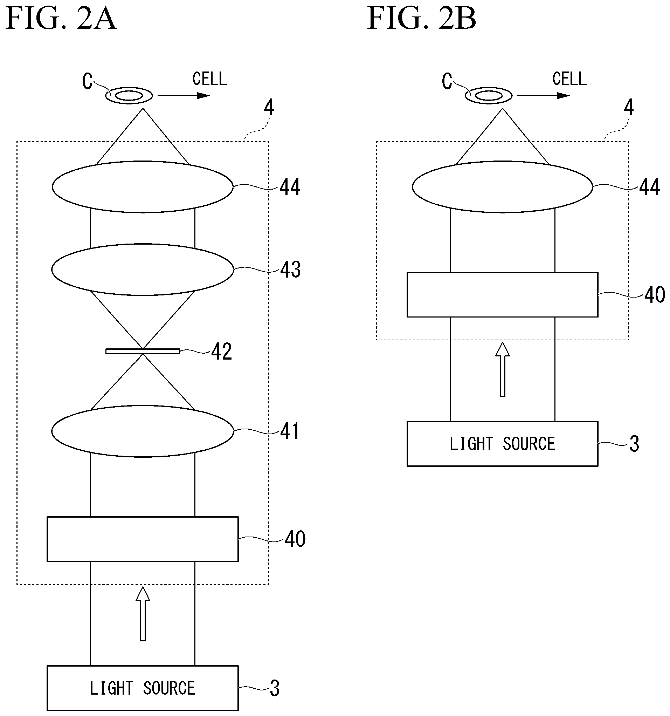

Here, the spatial light modulation unit 4 will be described with reference to FIG. 2. FIG. 2 is a diagram illustrating an example of the spatial light modulation unit 4 according to the present embodiment. The spatial light modulation unit 4 is an optical system including a spatial light modulator 40. FIG. 2(A) is a diagram illustrating an example of the spatial light modulation unit 4 according to the present embodiment. In FIG. 2(A), the spatial light modulation unit 4 includes the spatial light modulator 40, a first lens 41, a spatial filter 42, a second lens 43, and an objective lens 44. In the spatial light modulation unit 4, the spatial light modulator 40, the first lens 41, the spatial filter 42, the second lens 43, and the objective lens 44 are disposed on an optical path between the light source 3 and the light detector 6 in that order from the side closer to the light source 3.

In the configuration of the spatial light modulation unit 4 shown in FIG. 2, in order to accurately acquire spatial information of the object to be measured at a high resolution that enables image reconstruction or the like, it is preferable that the spatial light modulator 40 be disposed on the pupil plane of the objective lens 44 and disposed so that the object to be measured C1 passes through the focal position of the objective lens 44. In general objective lenses, their pupil planes are often located inside the objective lens housing. In such case where the pupil plane of the objective lens is located inside the objective lens housing, as shown in FIG. 2(A), it can be configured such that relay lenses (the first lens 41 and the second lens 43) are used to form an image of the pupil plane outside the objective lens housing, and that the spatial light modulator 40 is disposed at the position of the pupil plane.

On the other hand, in FIG. 2(B), the spatial light modulation unit 4 is configured with the relay lenses omitted. In this configuration, the spatial light modulation unit 4 is provided with the spatial light modulator 40 and the objective lens 44. The spatial light modulation unit 4 can also be configured with the relay lenses omitted as shown in FIG. 2(B), in which case the illumination optical system can be simplified and the device can be made smaller. Meanwhile, in this simplified configuration, it is preferable that the spatial light modulator 40 be installed close to the objective lens 44.

The spatial light modulator 40 structures incident light. Structuring incident light involves changing the optical characteristics of the incident light for each of a plurality of regions included in the incident surface of the incident light. The spatial light modulator 40 is an optical element that changes the optical characteristics of the incident light, which structures the illumination light LE1 and converts it into the structured illumination light SLE1. The surface of the spatial light modulator 40 on which the light is incident has a plurality of regions, and the optical characteristics of the illumination light LE1 are individually converted in each of the plurality of regions of the spatial light modulator 40 through which it passes. That is, in the light that has passed through the spatial light modulator 40, the optical characteristics of transmitted light change so as to be different from each other in a plurality of regions with respect to the optical characteristics of the incident light. Here, the optical characteristics are, for example, characteristics related to light in any one or more of intensity, wavelength, phase, and polarization state, but there is no limitation thereto. The spatial light modulator 40 changes the optical characteristics of the incident illumination light LE1 for each of the plurality of regions of the spatial light modulator 40, and makes it possible to irradiate the object to be measured C1 with the structured illumination light SLE1. This structured illumination light SLE1 is given a spatial distribution pattern of optical characteristics (for example, a spatial distribution of light intensity) based on a change in optical characteristics that occur in each of the plurality of regions. Meanwhile, the spatial distribution pattern of optical characteristics given to the structured illumination light SLE1 on the basis of a change in optical characteristics that occur in each of the plurality of regions is also referred to as a structured illumination pattern.

The spatial light modulator 40 includes, for example, a diffractive optical element (DOE), a spatial light modulator (SLM), and a digital micromirror device (DMD). In addition, as another example, the spatial light modulator 40 includes a film with a plurality of regions having different optical characteristics printed on its surface, a mask for structured detection in which a transmission unit that transmits light and a blocking unit that blocks light are disposed to exhibit a binary pattern, and the like. Meanwhile, in a case where the illumination light LE1 emitted by the light source 3 is incoherent light, the spatial light modulator 40 includes a DMD.

In the present embodiment, the spatial light modulator 40 is, as an example, a DOE which is an optical element that controls the diffraction phenomenon of light using a formed fine shape. Here, the light is the illumination light LE1.

In the process of the object to be measured C1 passing through a light irradiation region to which the structured illumination light SLE1 is irradiated, the structured illumination light SLE1 generates electromagnetic waves which are modulated by the object being measured C1. The electromagnetic waves modulated by the object to be measured C1 are, for example, scattered light. In other words, the scattered light is scattered light derived from the structured illumination light SLE1. The scattered light is an example of electromagnetic waves modulated by the object to be measured C1 which is present in a light irradiation region irradiated with illumination light from the light source 3.

Examples of the scattered light derived from the structured illumination light SLE1 include light scattered in a forward direction (forward scattered light), light scattered in a backward direction (backward scattered light), and light scattered in a side direction (side scattered light). Here, the forward direction is a direction of the course of the illumination light with which the object to be measured C1 is irradiated. The backward direction is a direction opposite to the direction of the course of the illumination light with which the object to be measured C1 is irradiated. The side direction is a lateral direction of the course of the illumination light with which the object to be measured C1 is irradiated, and includes a wide range of directions other than the forward direction and the backward direction.

Another example of the modulated electromagnetic waves from the object to be measured C1 includes fluorescence emitted by fluorescent molecules being excited by the structured illumination light SLE1 or transmitted light obtained by the structured illumination light SLE1 being transmitted through the object to be measured C1, in the process of the object to be measured C1 passing through a light irradiation region which is irradiated with the structured illumination light SLE1. The modulated electromagnetic waves from the object to be measured C1 are detected by the light detector 6 as signal light.

Hereinafter, a case where the modulated electromagnetic waves from the object to be measured C1 is scattered light will be described as an example. Meanwhile, the method of calculating feature values which will be described in the following description can also be applied in the same way in a case where the modulated electromagnetic waves are fluorescence or transmitted light.

The optical system for light detection 5 is an optical mechanism for collecting scattered signal light LS1 from the object to be measured C1 onto the light detector 6, and includes an imaging lens (not shown).

The scattered light derived from the structured illumination light SLE1 is detected as the scattered signal light LS1 by light detectors installed in various directions. For example, FIG. 1 shows an example in which the scattered signal light LS1 is detected by the light detector 6 installed in the forward direction. Hereinafter, in a case that the scattered signal light LS1 derived from the structured illumination light SLE1 is acquired by the light detector installed forward, the signal light is referred to as a forward detected scattered light signal (SCfd: scattered light detected by forward detector). In addition, in a case that the scattered signal light LS1 is detected by the light detector installed in the backward direction, the signal light is referred to as a backward detected scattered light signal (SCbd: scattered light detected by backward detector). In addition, in a case that the scattered signal light LS1 is detected by the light detector installed in the side direction, the signal light is referred to as a side detected scattered light signal (SCsd: scattered light detected by side detector). The scattered signal light LS1 is signal light in a case where the modulated electromagnetic waves derived from the structured illumination light SLE1 are scattered light. The scattered signal light LS1 includes

morphological information of the object to be measured which is acquired using GC technology. Meanwhile, the morphological information of the object to be measured which is acquired using GC technology includes information relating to the internal structure of the object to be measured (such as the structure and arrangement of cell organelles in a case where the object to be measured is a cell) in addition to the shape of the object to be measured.

SCfd and SCbd may each be mixed with components corresponding to scattered light in other directions. As will be described later, the flow cytometer 1 removes the components corresponding to scattered light in other directions from SCfd and SCbd through analysis using the PC 8.

Meanwhile, in the following description, the signal light obtained by detecting the scattered light emitted from the object to be measured C1 when irradiation light such as laser light is irradiated without structuring is distinguished from the signal light obtained by detecting the scattered light derived from the structured illumination light SLE1 described above. In a flow cytometer of the related art, for example, scattered light scattered in approximately the same direction as the incident light can be detected as forward scattered light by a detector installed in front of the incident light. Similarly, scattered light installed on the side or back can be detected as side scattered light or backward scattered light by a detector installed in each direction. For the scattered light detected by irradiating the object to be measured C1 with unstructured coherent light, the total amount of intensity of forward scattered light is referred to as forward scattered light intensity (forward scatter: FSC), and the total amount of intensity of backward scattered light is referred to as backward scattered light intensity (backward scatter: BSC).

FSC and BSC have been conventionally used as indices for providing rough information about the size and internal structure of particles in measurements using a flow cytometer. Meanwhile, hereinafter, irradiating the object to be measured C1 with illumination light without structuring is also referred to as irradiation as spot light.

An imaging lens (not shown in FIG. 1) included in the optical system for light detection 5 focuses the scattered signal light LS1 from the object to be measured C1 onto the position of the light detector 6. Meanwhile, the imaging lens does not need to form an image insofar as it collects the scattered signal light LS1 from the object to be measured C1 onto the position of the light detector 6, but is more preferably disposed at a position where the scattered signal light LS1 is focused onto the position of the light detector 6. In addition, the optical system for light detection 5 may further include a dichroic mirror or a wavelength-selective filter in addition to the imaging lens.

The light detector 6 detects the scattered signal light LS1 collected by an imaging lens 50. Meanwhile, in FIG. 1, although an example in which the light detector 6 is disposed in the forward direction and scattered light is detected is described, the light detector 6 can also be installed at one or more positions in the backward direction or the side direction relative to the irradiation direction of the structured illumination light SLE1. A plurality of light detectors 6 may be installed. In that case, the plurality of the light detectors 6 are installed at two or more positions selected from the positions in the forward direction, backward direction, or side direction.

The scattered signal light LS 1 such as SCfd and SCbd detected in the flow cytometer 1 is a signal in which the component identical to the component detected as FSC and BSC in a conventional flow cytometer is convolved and multiplied by the spatial distribution of optical characteristics (structured illumination pattern) included in the structured illumination light SLE1 and the shape of the object to be measured C 1. Therefore, when the waveform of the scattered signal light LS 1 is averaged (integrated), shape-derived information is averaged, and a feature value in which components corresponding to FSC and BSC are strongly reflected can be obtained.

Here, the light detector 6 detects the scattered signal light LS 1 and converts it into an electrical signal. The light detector 6 is a photomultiplier tube (PMT) as an example. The light detector 6 detects the intensity of the scattered signal light LS 1 collected by the imaging lens 50 in a time series. As described above, the scattered signal light LS1 is emitted from the object to be measured C1 flowing through the flow path 20 by irradiating the object to be measured C1 with the structured illumination light SLE1. That is, the light detector 6 detects, in a time series, the intensity of the scattered signal light LS1 emitted from the object to be measured C1 by irradiating the object to be measured C1 flowing through the flow path 20 with the structured illumination light SLE1. The light detector 6 may be a single sensor composed of a single light receiving element, or may be a multi sensor composed of a plurality of light receiving elements.

The DAQ device 7 converts the electrical signal pulse output by the light detector 6 into electronic data for each pulse. The electronic data includes a set of time and intensity of the electrical signal pulse. The DAQ device 7 is an oscilloscope as an example.

The PC 8 is a device which is included in the flow cytometer 1 and performs information processing. The flow cytometer 1 discriminates the object to be measured C1 on the basis of the result of information processing by the PC 8. The information processing includes a process of calculating the feature value of the object to be measured C1. The PC 8 calculates the feature value indicating the optical characteristics of the object to be measured C1 on the basis of the electronic data output from the DAQ device 7. The electronic data output from the DAQ device 7 is referred to as signal information D1. The signal information D1 indicates a temporal change in the intensity of the scattered signal light LS1. That is, the signal information D1 indicates a temporal change in the intensity of the electromagnetic waves obtained by receiving the electromagnetic waves (scattered light as an example) from the object to be measured C1 which is present in a light irradiation region irradiated with light from the light source 3 and converting it into electronic data. The temporal change in the intensity of the electromagnetic waves is also referred to as a change in the intensity of the electromagnetic waves over time. The signal information D1 which is electronic data output from the DAQ device 7 is an example of signal information indicating a temporal change in the intensity of electromagnetic waves. The PC 8 is an example of a feature value calculation device. The specific amount calculation device is a specific amount calculation device that calculates a feature value indicating the optical characteristics of an object to be measured in a flow cytometry method.

The PC 8 acquires the signal information D1 from the DAQ device 7 and generates waveform data based on the acquired signal information D1. In the present embodiment, as an example, the waveform data based on the signal information D1 is time waveform data W1 which is waveform data indicating a temporal change in the intensity of electromagnetic waves.

Meanwhile, the signal information D1 indicating a temporal change in the intensity of the scattered signal light LS1 can be used by further converting the waveform data into a frequency spectrum. The waveform data obtained by converting the horizontal axis of the waveform data indicating a temporal change in the intensity of electromagnetic waves to the frequency is referred to as spectral waveform data W2. An example of the spectral waveform data W2 is data in which the signal intensity obtained by performing Fourier transformation on the signal information D1 indicating a temporal change in the intensity of electromagnetic waves is shown with respect to frequency.

In the present embodiment, an example of a case where the scattered signal light LS1 is detected using a configuration for structured illumination in the flow cytometer 1 is described, but there is no limitation thereto. The scattered signal light LS1 may be detected using a configuration for structured detection in the flow cytometer 1.

As described above, in the configuration for structured illumination, the spatial light modulation unit 4 is provided between the light source 3 and the flow path 20 through which the object to be measured C1 flows. On the other hand, in the configuration for structured detection, a spatial light modulation unit including a DMD or the like is provided between the flow path 20 and the light detector 6. Here, the spatial light modulation unit 4 is provided between the flow path 20 and the optical system for light detection 5. In the configuration for structured detection, the spatial light modulator 40 included in the spatial light modulation unit 4 can also be the same as the spatial light modulator 40 that can be used in the configuration for structured illumination, but a film including a plurality of regions having different optical characteristics, or a mask for structured detection in which a transmission unit that transmits light and a blocking unit that blocks light are disposed to exhibit a binary pattern, is suitable.

In the configuration for structured detection, the object to be measured C1 is irradiated with the unstructured illumination light LE1 from the light source 3. The scattered light generated by the illumination light LE1 being scattered by the object to be measured C1 is structured by the spatial light modulation unit 4. The light detector 6 detects the light in which the structured scattered light is collected by the optical system for light detection 5 as scattered signal light.

[Configuration of feature value calculation device]

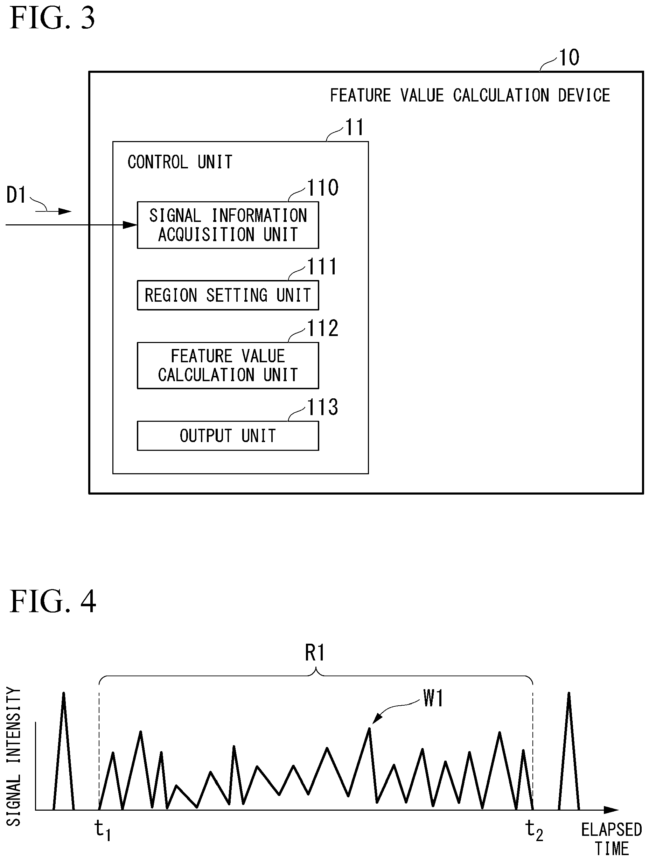

FIG. 3 is a diagram illustrating an example of a configuration of a feature value calculation device 10 according to the present embodiment. The feature value calculation device 10 is a feature value calculation device that calculates a feature value indicating the optical characteristics of an object to be measured using a flow cytometry method. The feature value calculation device 10 includes a control unit 11. The control unit 11 includes, for example, a central processing unit (CPU), a graphics processing unit (GPU), a field-programmable gate array (FPGA), or the like, performs various calculations, and exchange information. Each functional unit included in the control unit 11 is realized by the CPU reading a program from a read only memory (ROM) and executing processing. Each functional unit included in the control unit 11 can also be realized by the CPU reading a program from a hard disk and executing processing. In addition, the program which is executed by the control unit 11 can also be read from an external device (not shown) and executed.

Meanwhile, in a case where morphological information of the object to be measured is acquired from waveform data indicating a temporal change in the intensity of electromagnetic waves in the flow cytometer 1, some or all of the components of the feature value calculation device 10 according to the present embodiment may be shared with a device for acquiring the morphological information.

The control unit 11 includes a signal information acquisition unit 110, a region setting unit 111, a feature value calculation unit 112, and an output unit 113.

The signal information acquisition unit 110 acquires the signal information D1 from the DAQ device 7.

The region setting unit 111 sets a region for calculating a feature value on the basis of the signal information D1 acquired by the signal information acquisition unit 110. The feature value is calculated in the region set in the time waveform data W1, wherein the temporal change in the intensity of electromagnetic waves indicated by the signal information D1 acquired by the signal information acquisition unit 110 is used, as it is, as the time waveform data W1.

The feature value calculation unit 112 calculates a feature value indicating the optical characteristics of the object to be measured C1 from the time waveform data W1 in the region set by the region setting unit 111. The feature value calculated from the time waveform data W1 is the average value, maximum value, minimum value, median value, mode value, or integrated value of the optical signal intensity as an example, but there is no limitation thereto. In addition to values directly calculated from the time waveform data W1, the feature value may be calculated by combining numerical values calculated from the time waveform data W1 as in an example to be described later. The feature value is a numerical value indicating one optical feature of the object to be measured which is calculated from the time waveform data W1. The numerical value changes depending on the spatial distribution of optical characteristics (structured illumination pattern) included in the structured illumination light SLE1 to be radiated and the region set as a processing target (time domain, or frequency domain in a case where the waveform data is the spectral waveform data W2). The numerical value is an index indicating the optical characteristics of the object to be measured when the spatial distribution of optical characteristics (structured illumination pattern) included in the structured illumination light SLE1 and the conditions for the set region are met.

Meanwhile, in the above, a case where the region setting unit 111 uses a temporal change in the intensity of electromagnetic waves indicated by the signal information D1 acquired by the signal information acquisition unit 110 is used, as it is, as the time waveform data W1 has been described. However, the signal information D1 acquired by the signal information acquisition unit 110 can also be used by converting it into waveform data with domain other than the elapsed time. The region setting unit 111, for example, can also use the spectral waveform data W2 in which the signal information D1 acquired by the signal information acquisition unit 110 is shown as a frequency spectrum as waveform data and set a region in which the feature value is calculated in the spectral waveform data W2. In that case, for example, the region setting unit 111 generates the spectral waveform data W2 obtained by performing Fourier transformation on the signal information D1.

The output unit 113 outputs the feature value calculated by the feature value calculation unit 112 to an external device (not shown). The external device is, for example, a display such as a display device, a storage device included in a server, or the like. Meanwhile, the output unit 113 may output the calculated feature value to a display device or a storage device of its own device. In that case, the feature value calculation device 10 has a display device and a storage device (not shown) in addition to the control unit 11.

[Signal information]

FIG. 4 is a diagram illustrating an example of the time waveform data W1 according to the present embodiment which is waveform data in cases where the elapsed time is defined as a domain. The time waveform data W1 shown as a graph in FIG. 4 indicates the intensity (signal intensity) of electromagnetic waves obtained as electronic data when the electromagnetic waves from the object to be measured C1 are received, with respect to the time that has elapsed since measurement performed by the flow cytometer 1 was started (elapsed time). The time waveform data W1 is data indicating the intensity of electromagnetic waves with the elapsed time as a domain.

A region R1, which is set in the waveform data by the region setting unit 111, is a region to be processed in which the feature value is calculated. In the example of FIG. 4, the region R1 indicates a region from time t1 to time t2 in the elapsed time. The region setting unit 111 sets, for example, a region of the time waveform data W1 from time t1 at which electromagnetic waves emitted from the object to be measured C1 by structured illumination light is first detected to time t2 at which the electromagnetic waves are last detected as the region R1. That is, the feature value is calculated in the region R1 of the waveform data set by the region setting unit 111.

In the present embodiment, as described above, the data indicating a temporal change in the intensity of electromagnetic waves indicated by the signal information D1 can also be further displayed as a frequency spectrum and used as the spectral waveform data W2.

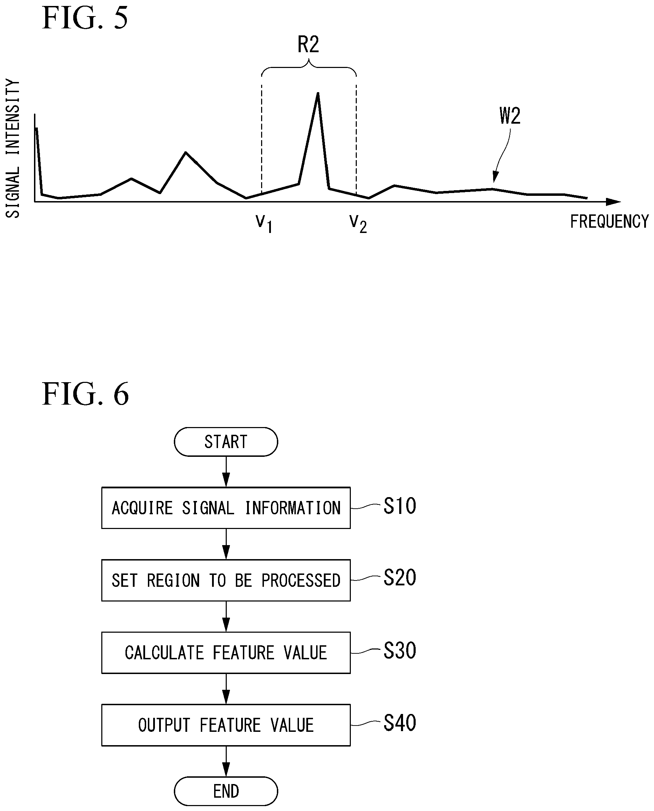

FIG. 5 is a diagram illustrating an example of the spectral waveform data W2 according to the present embodiment which is waveform data in cases where the frequency is defined as a domain. The spectral waveform data W2 shown as a graph in FIG. 5 is waveform data obtained by receiving electromagnetic waves from the object to be measured C1 and performing Fourier transformation on the signal information D1 indicating a change in the intensity of the electromagnetic waves obtained as electronic data over time. In the spectral waveform data W2, the intensity of a signal is shown as a frequency spectrum with respect to frequency. The spectral waveform data W2 is data indicating a spectral signal intensity with frequency as a domain.

A region R2 is an example of a frequency region set by the region setting unit 111 to be processed in which a feature value is calculated. In the example of FIG. 5, the region R2 indicates a frequency region from frequency v1 to frequency v2 in the frequency. The region setting unit 111 sets, for example, a region of a predetermined frequency range in the waveform data of a frequency spectrum with frequency as a domain as the region R2. The feature value calculation unit 112 calculates, for example, the maximum value from the frequency spectrum in the region set by the region setting unit 111 as a feature value indicating the characteristics of the object to be measured C1.

As another example, the feature value calculation unit 112 sets a specific region around a desired frequency in the waveform data of a frequency spectrum as the region R2, and the region setting unit 111 calculates the integrated value of signal intensity in the set region R2 as a feature value. In this case, the calculated feature value is calculated as a feature value indicating the structural feature of the object to be measured C1 which is extracted in the specific frequency region.

[Feature value calculation process]

FIG. 6 is a diagram illustrating an example of a feature value calculation process according to the present embodiment. The feature value calculation process is executed by the control unit 11 of the feature value calculation device 10. Meanwhile, the following description relates to a case where the time waveform data W1 is used as waveform data based on the signal information D1, but the same applies to a case where the spectral waveform data W2 is used as waveform data.

Step S10: The signal information acquisition unit 110 acquires the signal information D1 from the DAQ device 7. The signal information acquisition unit 110 supplies the acquired signal information D1 to the region setting unit 111.

Step S20: On the basis of the signal information D1 acquired by the signal information acquisition unit 110, the region setting unit 111 sets a domain of the waveform data for which a feature value is calculated, and sets a region to be processed in which the feature value is calculated in the waveform data for calculating the feature value. The region setting unit 111 supplies information indicating the set region to the feature value calculation unit 112. For example, in a case where the domain of the waveform data for calculating a feature value is set to elapsed time, the region setting unit 111 supplies the feature value calculation unit 112 with information indicating a region for which the feature value of the time waveform data W1 is to be calculated.

Step S30: The feature value calculation unit 112 calculates a feature value indicating the optical characteristics of the object to be measured from the signal intensity for the region set by the region setting unit 111. For example, in a case where the domain of the waveform data for calculating a feature value is set to elapsed time, the feature value calculation unit 112 calculates the average value of the signal intensity with respect to time for the region of time set by the region setting unit 111. In addition, in another example, the feature value calculation unit 112 calculates the integrated value of the signal intensity by integrating the waveform data indicated by the signal information D1.

As described above, in the scattered signal light LS1, such as SCfd and SCbd, scattered light components of other directions ares mixed. By removing scattered light components of other directions from the scattered signal light LS1, the feature value related to scattering in a specific direction can be extracted as an amount in which the feature of scattering in the specific direction is further reflected.

For example, a large quantity of FSC components and BSC components are mixed in with SCfd. By removing the BSC component from SCfd, the feature value related to scattering to the forward direction can be extracted as an amount in which the feature of forward scattering is further reflected.

On the other hand, in SCbd, the proportion of FSC components mixed is small, and SCbd and BSC are highly correlated.

Meanwhile, the feature value calculation unit 112 may combine a plurality of calculated feature values to form a new feature value. For example, the maximum value, the minimum value, and the like normalized by the average value may be used as the new feature value.

In addition, insofar as the scattered signal light LS1 caused by the same the object to be measured C1, the feature value calculation unit 112 may combine the feature values calculated from the scattered signal light detected in different directions to form a new feature value. Here, the scattered signal light LS1 caused by the same the object to be measured C1 is signal light detected for the same object to be measured at the same time. For example, SCfd and SCbd may be measured simultaneously for the same object to be measured, and the feature value derived from backward scattering and the feature value derived from forward scattering may be combined to form a new feature value.

Meanwhile, in a case where the waveform data (especially SCfd) of the scattered signal light LS 1 due to scattering to a certain direction is mixed with components corresponding to scattered light of other directions, it is preferable to perform correction for removing the influence of the mixed components in order to accurately acquire only the characteristics of the object to be measured which are reflected in the light scattered to a specific direction.

Step S40: The output unit 113 outputs the feature value calculated by the feature value calculation unit 112 to an external device.

With the above, the control unit 11 completes the feature value calculation process.

[Example 1]

Here, Example 1 of the present embodiment will be described. The flow cytometer according to the present example is referred to as a flow cytometer FCM1. The flow cytometer FCM1 is a flow cytometer based on GC technology. In the present example, commercially available blood cell-mimicking beads (WBC Scatter mimic Cat No. SSB-04-C manufactured by Slingshot Biosciences) were used as the object to be measured C1.

In the flow cytometer FCM1, laser light (405 nm) was structured as the illumination light LE1 from the light source 3 and radiated onto the object to be measured C1 passing through the flow path 20. And, the scattered signal light LS1 emitted from the object to be measured C1 was detected by the light detector 6 installed forward and backward to acquire waveform data indicating a temporal change in the intensity of the scattered signal light LS1.

In addition, in the flow cytometer FCM1, FSC and BSC were measured simultaneously using a separate measurement channel for the purpose of comparison. In the measurement of FSC and BSC of the flow cytometer FCM1, the illumination light LE1 from the light source 3 was radiated onto the object to be measured C1 as spot light without being structured, and forward and backward scattered light was detected. As the illumination light LE1 from the light source 3, laser light (637 nm) was used. FSC and BSC are the total amount of intensity of scattered light which is also measured in a conventional flow cytometer.

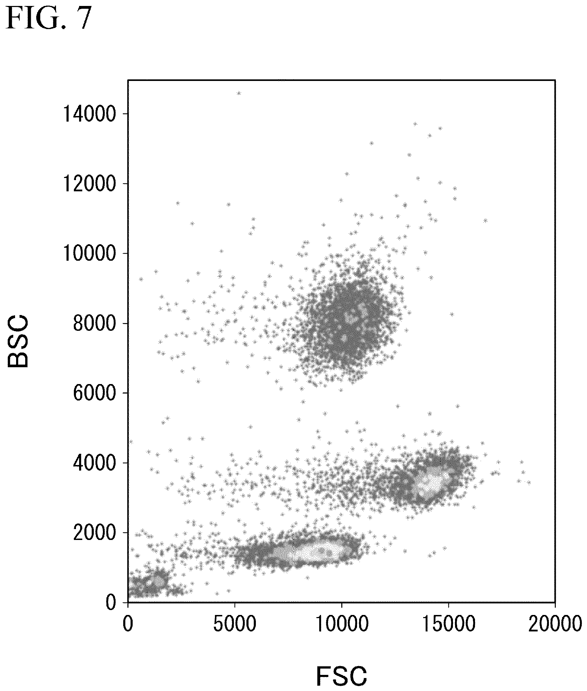

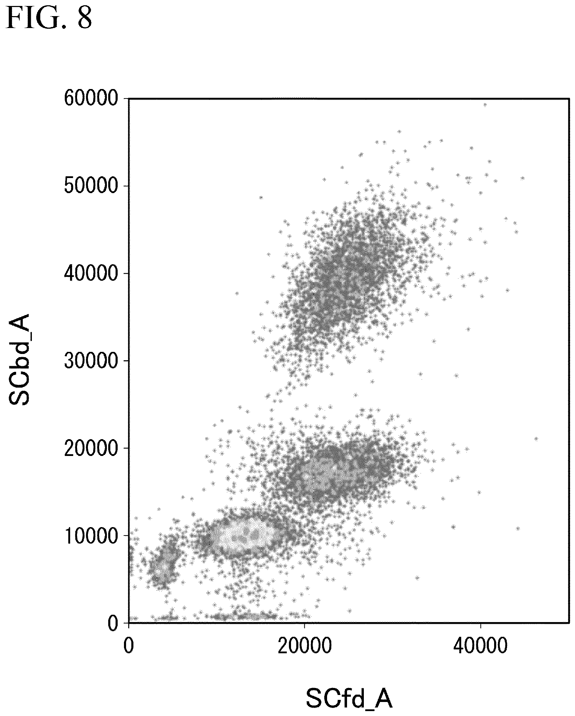

FIG. 7 is a diagram illustrating a correlation between FSC and BSC detected by the flow cytometer FCM1 according to the present example. FIG. 8 is a diagram illustrating a correlation between the integrated value SCfd_A of SCfd and the integrated value SCbd_A of SCbd detected by the flow cytometer FCM1 according to the present example. The correlation between FSC and BSC shown in FIG. 7 is shown for comparison with the correlation between the integrated value SCfd_A and the integrated value SCbd_A shown in FIG. 8.

Each of a plurality of plots shown in FIGS. 7 and 8 indicates the measurement results for each of a plurality of objects to be measured C1.

Here, the integrated value SCfd_A is a feature value calculated by integrating the waveform data indicating a temporal change in a scattered light signal which is detected by a light detector installed forward when the object to be measured C1 is irradiated with structured illumination light. In addition, the integrated value SCbd_A is a feature value calculated by integrating the waveform data indicating a temporal change in the scattered signal light which is emitted from the object to be measured C1 and is detected by a light detector installed backward.

Comparing the correlation between FSC and BSC indicated by the measurement results in FIG. 7 and the correlation between the integrated value SCbd_A and the integrated value SCbd_A indicated by the measurement results in FIG. 8, it has been confirmed that there is a similar correlation between the two, and it has been shown that these feature values serve as indices indicating optical characteristics related to forward or backward scattering of the object to be measured C1.

As described above, the feature value calculation device 10 according to the present embodiment is a feature value calculation device that calculates the optical characteristics of an object to be measured using a flow cytometry method, and includes the signal information acquisition unit 110, the region setting unit 111, and the feature value calculation unit 112.

The signal information acquisition unit 110, by using any one or more of a configuration for structured illumination or a configuration for structured detection, acquires the signal information D1 indicating a temporal change in the intensity of the electromagnetic waves (the scattered signal light LS1 in the present embodiment) generated by the light detector 6 receiving the modulated electromagnetic waves (the scattered signal light LS1 in the present embodiment) from the object to be measured C1 which is present in a light irradiation region irradiated with light from the light source 3. In the configuration for structured illumination, the illumination light from the light source 3 is structured as structured illumination light and radiated onto the object to be measured C 1. In the configuration for structured detection, the electromagnetic waves from the object to be measured C1 are structured and detected.

From the signal information D1 acquired by the signal information acquisition unit 110, the region setting unit 111 sets a domain (elapsed time in the present embodiment) of the waveform data (the time waveform data W1 in the present embodiment) in which the feature value is calculated and a region to be processed in which the feature value is calculated.

The feature value calculation unit 112 calculates the feature value indicating the optical characteristics of the object to be measured C1 from the waveform data (the time waveform data W1 in the present embodiment) in the region set by the region setting unit 111. In other words, the feature value calculation unit 112 calculates the feature value indicating the optical characteristics of the object to be measured C1 using the waveform data of the region portion set by the region setting unit 111.

With such a configuration, the feature value calculation device 10 according to the present embodiment can calculate the feature value indicating the optical characteristics of the object to be measured C1 from the waveform data (the time waveform data W1 in the present embodiment) based on the signal information D1 indicating a temporal change in the intensity of the electromagnetic waves from the object to be measured C1, and thus can acquire various types of information included in the optical signal detected in GC technology.

The GC technology is known as a technique that makes it possible to acquire detailed morphological information of cells with higher resolution than conventional flow cytometry. Until now, in the flow cytometer using tGC technology, the waveform data indicating a temporal change in an optical signal including morphological information with high resolution has been used, for example, for a discrimination process in which an object to be measured is predicted on the basis of a discrimination model created through machine learning. On the other hand, the flow cytometer 1 according to the present embodiment not only uses the waveform data of an optical signal acquired through GC technology using structured illumination light as information including morphological information of the object to be measured with high resolution, but makes it possible to newly extract information relating to the total amount of scattered light intensity which has been used in a conventional flow cytometer by calculating a feature value from the waveform data.

Here, in the flow cytometer 1 according to the present embodiment, there is no need to newly acquire waveform data indicating a temporal change in an optical signal in order to calculate a feature value, and the waveform data acquired using GC technology for the discrimination process based on morphological information can be used as it is. Therefore, there is no need to change its optical system such as installing a new detector in order to calculate a feature value. Therefore, in the flow cytometer 1 according to the present embodiment, information that can be associated with information relating to the scattered light intensity acquired in a conventional flow cytometer (feature value calculated from waveform data indicating a change in the scattered signal light LS 1 over time) can be easily acquired simultaneously with morphological information with high resolution using GC technology without setting a new optical system.

In the flow cytometer 1 according to the present embodiment, measurement for calculating the feature value indicating the optical characteristics of the object to be measured C1 may be executed in parallel to measurement for performing discrimination based on morphological information of the object to be measured C1 from the waveform data using a discrimination model created in advance by learning a relationship between the waveform data and the morphological information of the object to be measured C1 through machine learning. For example, in the flow cytometer 1 according to the present embodiment, machine learning is performed using training data including waveform data of the object to be measured C1 acquired using GC technology and information for specifying the object to be measured C1 in association with each other, and the object to be measured C1 is discriminated using the waveform data of the object to be measured C1 on the basis of the discrimination model created in advance. However, the waveform data detected for discriminating the object to be measured C1 can also be used to calculate the feature value indicating the optical characteristics of the object to be measured C1.

Further, the flow cytometer 1 according to the present embodiment may be configured to be able to simultaneously acquire both the scattered signal light LS 1 (GC waveform) emitted from the object to be measured C1 irradiated with structured illumination light and the conventional scattered light intensity (FSC, BSC, SSC). For example, the flow cytometer 1 according to the present embodiment can be configured to have both a configuration in which scattered light emitted from the object to be measured C1 is detected as a GC waveform by means of structured illumination light and a configuration in which it is detected as scattered light by means of unstructured spot light. In that case, when the scattered signal light LS1 (GC waveform) derived from structured illumination light and the conventional scattered light intensity are simultaneously measured using standard beads for accuracy control or the like in a flow cytometer, the correlation between the feature value converted from the waveform data indicating the scattered signal light LS 1 (GC waveform) and the actual measured value of the conventional scattered light intensity can be further enhanced using, for example, a linear regression equation or the like. Thereby, it is possible to organize information relating to the conventional scattered light intensity which has been accumulated in the conventional flow cytometers and the measurement results of the feature value converted from GC waveform data of a flow cytometer using GC technology such as the flow cytometer 1 according to the present embodiment in a more precise association with each other.

In addition, in the feature value calculation device 10 according to the present embodiment, the waveform data based on the signal information D1 is not limited to time-series waveform data (the time waveform data W1) of an optical signal. For example, the region setting unit 111 may use the frequency spectrum obtained by performing Fourier transformation on the acquired scattered signal light LS 1 as waveform data (the spectral waveform data W2 with a domain as frequency), and calculate the feature value for a specific frequency range set as a region in the spectral waveform data W2.

With such a configuration, in the feature value calculation device 10 according to the present embodiment, an optical feature for a specific frequency of the object to be measured C1 can be extracted from a frequency spectrum to be calculated as a feature value., Thus, for example, structural information of the object to be measured C1 extracted in a specific frequency region can be acquired among the various types of information included in the scattered signal light LS1.

In addition, in the feature value calculation device 10 according to the present embodiment, the signal information acquisition unit 110 acquires signal information indicating a temporal change in the intensity of scattered light from the object to be measured C1 which is present in a light irradiation region as the signal information D1. The waveform data based on the signal information D1 is data indicating the intensity of scattered light with elapsed time as a domain (the time waveform data W1 in the present embodiment). The region setting unit 111 sets a region in a range of a specific elapsed time as the region. The feature value calculation unit 112 calculates, as a feature value, the integrated value of the intensity of scattered light (the integrated value SCfd_A and the integrated value SCbd_A in the present embodiment) received by the light detector 6 in a range of a specific elapsed time from the waveform data (the time waveform data W1 in the present embodiment) in the region set by the region setting unit 111.

With such a configuration, the feature value calculation device 10 according to the present embodiment can calculate the integrated value of the intensity of scattered light (the integrated value SCfd_A and the integrated value SCbd_A in the present embodiment) as a feature value. Therefore, it can measure an index indicating optical characteristics related to the intensity of forward or backward scattering of the object to be measured C1 as one of various types of information included in the optical signal detected in GC technology.

(Second embodiment)

Hereinafter, a second embodiment of the present invention will be described in detail with reference to the accompanying drawings.

In the above first embodiment, a case where a feature value indicating the optical characteristics of the object to be measured is calculated from waveform data indicating a temporal change in optical signal intensity has been described. The feature value obtained in this way is an index indicating the optical characteristics of the object to be measured (for example, an index indicating the intensity of scattering to a certain direction). However, it cannot be replaced with the numerical value of the scattered light intensity (FSC, SSC, BSC) acquired in a conventional flow cytometer as it is. Therefore, in the present embodiment, a case where the calculated feature value is converted into a value corresponding to the scattered light intensity acquired in a conventional flow cytometer will be described.

The feature values converted into values corresponding to FSC, SSC, and BSC according to the present embodiment are also one of the feature values in the present invention. In addition, in the present embodiment, the scattered light is an example of electromagnetic waves emitted from the object to be measured in a case where the object to be measured is irradiated with structured illumination light.

The flow cytometer according to the present embodiment is referred to as a flow cytometer 1a, and the feature value calculation device is referred to as a feature value calculation device 10a. The configuration of the flow cytometer 1a is the same as the configuration of the flow cytometer 1 (FIG. 1) except that the feature value calculation device 10a is provided as the PC 8.

Meanwhile, the same components as in the above-described first embodiment are denoted by the same reference numerals and signs, and the same components and operations may not be described.

[Configuration of feature value calculation device]

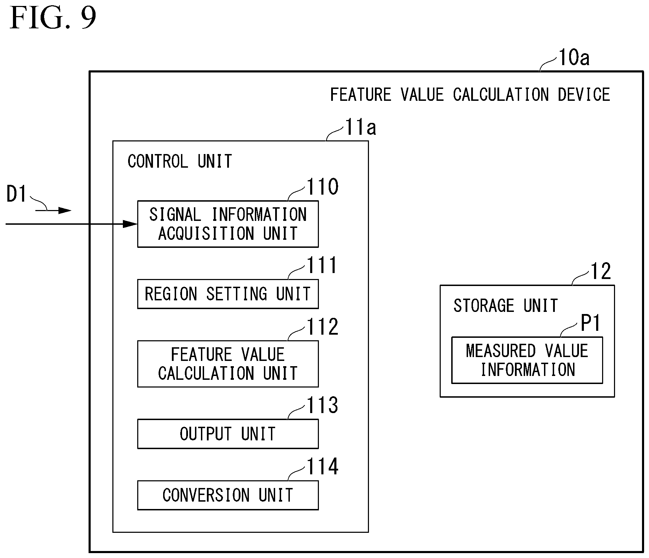

FIG. 9 is a diagram illustrating an example of a configuration of the feature value calculation device 10a according to the present embodiment. The feature value calculation device 10a includes a control unit 11a and a storage unit 12.

The control unit 11a includes the signal information acquisition unit 110, the region setting unit 111, the feature value calculation unit 112, the output unit 113, and a conversion unit 114.

The conversion unit 114 converts the feature value calculated by the feature value calculation unit 112 into a value corresponding to a measured value obtained by measurement in a conventional flow cytometer. Here, the measured value obtained by measurement in a conventional flow cytometer is, for example, a measured value which is used by measuring scattered light obtained by irradiation with unstructured spot light as scattered light intensity such as FSC or BSC. The conversion unit 114 converts, for example, the feature value calculated by the feature value calculation unit 112 for a reference object to be measured into a value corresponding to the scattered light intensity (total amount of scattered light intensity) such as FSC or BSC measured for the same object to be measured by a conventional flow cytometer. A reference object to be measured C2 is, for example, a standard bead for accuracy control in a flow cytometer (for example, Flow-Check Pro manufactured by Beckman Coulter), an imitation cell (for example, WBC Scatter mimic manufactured by Slingshot Biosciences), or the like. In addition, hereinafter, a measurement sample including the reference object to be measured C2 is also referred to as a standard sample.

The flow cytometer that is used to acquire scattered light intensity such as FSC or BSC for a reference object to be measured may be a conventional flow cytometer that performs optical measurement by irradiation with unstructured spot light. By using a separate detection channel from the optical measurement that acquires waveform information for acquiring morphological information, it may also be possible to perform both two optical measurements simultaneously and in parallel in the flow cytometer using the GC technology.

In the following description, a flow cytometer that performs optical measurement by the irradiation with unstructured spot light is referred to as a conventional flow cytometer. In addition, a case where optical measurement for acquiring a measured value such as FSC or BSC is performed by a conventional flow cytometer separately from a flow cytometer that acquires a feature value using GC technology will be mainly described.

The storage unit 12 stores various types of information. Various types of information include measured value information P1. The measured value information P1 is information including a measured value obtained by measurement of a reference object to be measured. The storage unit 12 is configured using a storage device such as a magnetic hard disk device or a semiconductor storage device.

[Feature value calculation process]

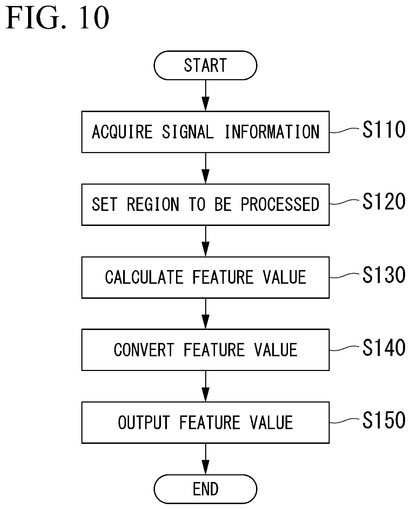

FIG. 10 is a diagram illustrating an example of a feature value calculation process according to the present embodiment. The feature value calculation process is executed by the control unit 11a of the feature value calculation device 10a.

Meanwhile, the processes of step S110, step S120, step S130, and step S150 are the same as the processes of step S10, step S20, step S30, and step S40 in FIG. 6, and thus the description thereof will be omitted below.

Meanwhile, although not shown in FIG. 10, before a feature value conversion process is executed in step S 140, a conventional flow cytometer is used in advance to perform measurement using a standard sample including the same object to be measured as the object to be measured C2 measured by the flow cytometer 1a and the measured value of the total amount of the intensity of scattered light (such as, for example, FSC and BSC) is acquired for the same object to be measured as the object to be measured C2.

Step S 140: The conversion unit 114 converts the feature value into a value corresponding to the total amount of scattered light acquired by a conventional flow cytometer other than the flow cytometer 1a on the basis of the feature value calculated by the feature value calculation unit 112 and the measured value obtained by measuring the reference object to be measured C2 with a conventional flow cytometer. For example, the conversion unit 114 converts the feature value calculated by the feature value calculation unit 112 into a value corresponding to the total amount of scattered light acquired by a conventional flow cytometer other than the flow cytometer 1a through a regression analysis based on the correlation between the measured value obtained by measuring the reference object to be measured C2 with a conventional flow cytometer and the feature value calculated by the feature value calculation unit 112.

The conversion unit 114 converts the feature value calculated by integrating SCfd calculated in step S130 into a value corresponding to FSC (the value is referred to as FSCest as will be described later), for example, on the basis of Expression (1). In addition, the conversion unit 114 converts the feature value calculated by integrating SCbd calculated in step S130 into a value corresponding to BSC (BSCest), for example, on the basis of Expression (2).

[Expression 1]

$$FSCest = [ SCfd + (SCbd + a) \times b + c ] \times d + e \qquad \cdots(1)$$

[Expression 2]

$$BSCest = (SCbd + a) \times b + c \qquad \cdots(2)$$

In this case, each of the parameters a to e in Expression (1) can be derived by associating, for example, the feature value calculated by integrating SCfd, the feature value calculated by integrating SCbd, and the measured value of FSC measured for the same object to be measured C2 using a conventional flow cytometer with Expression (1). In addition, each of the parameters a to c in Expression (2) can be determined by associating, for example, the feature value calculated by integrating SCbd with the measured value of BSC measured for the same object to be measured C2 using a conventional flow cytometer in Expression (2).

As another method, each parameter of Expressions (1) and (2) can be obtained by simultaneously fitting Expressions (1) and (2), for example, for a plot of the measured value of FSC against the integrated value of SCfd and a plot of the measured value of BSC against the integrated value of SCbd.

As still another method, the parameters from a to e can be obtained by a method in which the parameters a, b, and c in Expression (2) are acquired by fitting Expression (2) to a plot of the integrated value of SCbd and the measured value of BSC first, and the remaining parameters d and e in Expression (1) are additionally obtained by fitting Expression (1) to a plot of the integrated value of SCfd and the measured value of FSC.

As in the above example, for the reference object to be measured C2, by performing a regression analysis on the correlation between the measured value obtained by a conventional flow cytometer and the feature value calculated by the feature value calculation unit 112, it is possible to obtain a regression equation and necessary parameters by which the feature value calculated by the feature value calculation unit 112 is converted into a value corresponding to the total amount of scattered light acquired by a conventional flow cytometer other than the flow cytometer 1a.

Meanwhile, in general, since SSC largely corresponds to BSC, it is similarly possible to obtain the conversion value SSCest corresponding to SSC from the integrated value of SCsd on the basis of Expression (2), by using SSC and the integrated value of SCsd (scattered light signal derived from structured illumination light acquired by a light detector installed at the side) instead of BSC and the integrated value of SCbd in the above method.

With the above, the control unit 11a completes the feature value calculation process.

In the feature value calculation device 10a, by combining the feature value of forward scattered light with an amount obtained by converting the feature value of backward (side) scattered light and the measured value obtained by measurement of a standard sample, it is possible to generate a feature value corresponding to the measured value of FSC or BSC acquired by a conventional flow cytometer that performs optical measurement by irradiation with unstructured spot light from the scattered light signal derived from structured illumination light.

Meanwhile, the configuration of the flow cytometer 1a has been described taking as an example a case where forward and backward scattered light is acquired in the present embodiment, but there is no limitation thereto. For forward and side scattered light, side and backward scattered light, or forward, side, and backward scattered light, the total amount of scattered light intensity may be acquired by a conventional flow cytometer, and the feature value may be acquired by the flow cytometer 1a using GC technology. Thereby, as in the present embodiment, from the scattered light signal derived from the structured illumination light, it is possible to generate a feature value corresponding to the measured value of the total amount of the scattered light intensity acquired by a conventional flow cytometer. Meanwhile, the conventional flow cytometer performs optical measurement by irradiation with spot light.

In addition, the flow cytometer 1a and the conventional flow cytometer not only differ in the amount to be measured between SCfd and SCbd, FSC, SSC, and BSC, but also may differ in the configuration of optical elements included in the measurement system. In that case, when the feature value measured by the flow cytometer 1a is converted into a value corresponding to the scattered light intensity acquired in a conventional flow cytometer, it is necessary to further correct influences caused by differences in the configuration of optical elements between flow cytometers.

[Example 2]

In the present example, the configuration of the flow cytometer FCM1 used for measurement and the measurement conditions are the same as in Example 1 described above. In the present example, in a flow cytometer using GC technology, measurements corresponding to the total amount of the scattered light intensity such as FSC measured by a conventional flow cytometer were performed in parallel using a channel different from the optical system that acquires waveform information from which morphological information can be acquired. In the present example, commercially available blood cell-mimicking beads (WBC Scatter mimic Cat No. SSB-04-C manufactured by Slingshot Biosciences) were used as the object to be measured C2.

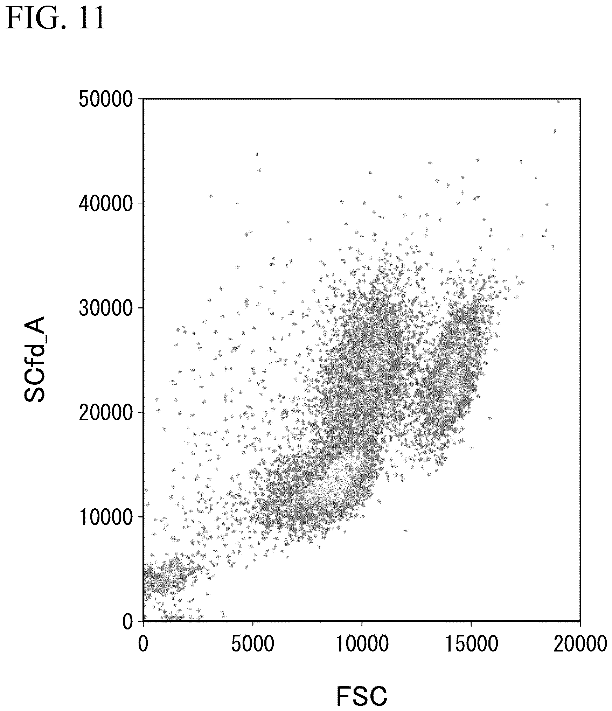

FIG. 11 is a diagram illustrating a correlation between the measured value of FSC according to the present example and the integrated value (the integrated value SCfd_A in an example of FIG. 11) which is one of the feature values of SCfd. In the present example, in order to remove components derived from backward scattered light included in SCfd, the feature value (the integrated value SCfd A) calculated on the basis of the measured value was further converted using the following Expression (3). Here, the converted integrated value of SCbd is also referred to as FSCest. Meanwhile, about Expression (3), a fitting was performed to above Expression (3) using the integrated value of SCfd (that is, the integrated value SCfd_A), the integrated value of SCbd (the integrated value SCbd_A), and the measured value of FSC to develop the regression equation shown in Expression (3).

[Expression 3]

$$SCfd, SCbd \rightarrow FSCest$$
$$FSCest = (SCfd + bsfsSlope*(SCbd + bsOffset) + bsfsOffset)*fsSlope + fsOffset$$

$fsOffset: 10103.273019361108$
$bsOffset: -43856.42648998313$
$bsfsOffset: -47651.56483380226$
$bsfsSlope: -0.4104063384916586$
$fsSlope: 0.2410252647875023$

$\cdots (3)$

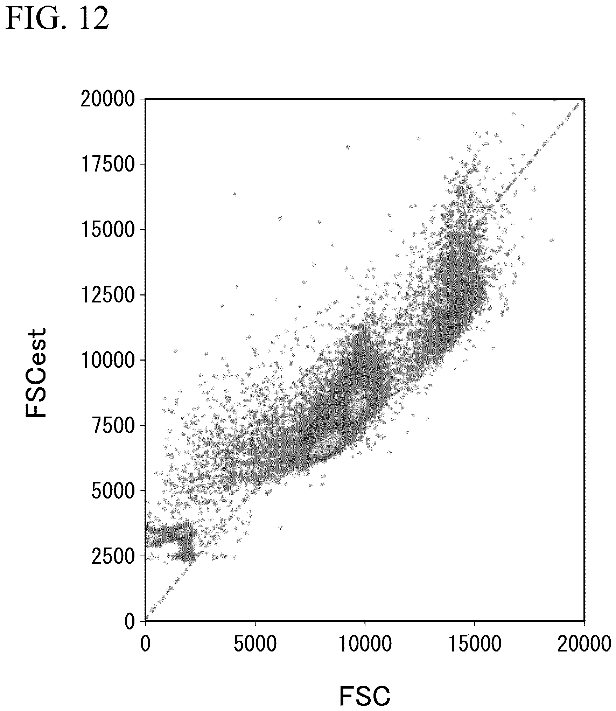

FIG. 12 is a diagram illustrating a correlation between the measured value of FSC measured by the flow cytometer FCM1 and the integrated value of SCfd (FSCest) converted from the integrated value SCfd_A and the integrated value SCbd_A using Expression (3). As shown in FIG. 12, it was confirmed that a better correlation of FSCest with FSC is shown when it is compared with the correlation of the feature value before conversion (the integrated value SCfd_A) with FSC as shown in FIG. 11. As for the correlation between FSCest and FSC, a value close to 1 is obtained as a correlation coefficient, and the integrated value of SCfd (the integrated value SCfd_A) is converted to a value more closely corresponds to FSC.

As described above, the feature value calculation device 10a according to the present embodiment includes the conversion unit 114.

The conversion unit 114 converts the feature value (for example, the integrated value SCfd_A in the present embodiment) into a value corresponding to the intensity of electromagnetic waves (for example, FSC in the present embodiment) acquired by a conventional flow cytometer on the basis of the feature value calculated by the feature value calculation unit 112 and the measured value obtained by measurement of the reference object to be measured C2.

With such a configuration, the feature value calculation device 10a according to the present embodiment can convert the calculated feature value into a value corresponding to the intensity of electromagnetic waves acquired by a conventional flow cytometer.

Meanwhile, some of the feature value calculation devices 10 and 10a in the above-described embodiment, for example, the signal information acquisition unit 110, the region setting unit 111, the feature value calculation unit 112, and the conversion unit 114 may be realized by a computer. In that case, a program for realizing this control function may be recorded in a computer readable recording medium, and each of the units may be realized by causing a computer system to read and execute the program recorded in this recording medium. Meanwhile, the term "computer system" referred to there is a computer system built into the feature value calculation devices 10 and 10a, and is assumed to include an OS and hardware such as peripheral devices. In addition, the term "computer readable recording medium" refers to a portable medium such as a flexible disk, a magnetooptic disc, a ROM, a CD-ROM, and a storage device such as a hard disk built into a computer system. Further, the "computer readable recording medium" may include recording mediums that dynamically hold a program during a short period of time like networks such as the Internet or communication lines when a program is transmitted through communication lines such as a telephone line, and recording mediums that hold a program for a certain period of time like a volatile memory inside a computer system serving as a server or a client in that case. In addition, the above-mentioned program may be a program which is used for realizing a portion of the aforementioned functions, and may be a program which is capable of realizing the aforementioned functions by a combination with programs previously recorded in the computer system.

In addition, some or all of the feature value calculation devices 10 and 10a in the above-described embodiment may be realized as an integrated circuit such as a large scale integration (LSI). Each functional block of the feature value calculation devices 10 and 10a may be individually formed as a processor, or some or all of the functional blocks may be integrated and be formed as processors. In addition, a method of forming an integrated circuit may be realized using a dedicated circuit or a general-purpose processor without being limited to an LSI. In addition, in a case where an integrated circuit technology substituting for LSI appears with the development of semiconductor technology, an integrated circuit based on the technique may be used.

Hereinbefore, although an embodiment of this invention has been described in detail with reference to the accompanying drawings, specific configurations are not limited to those described above, and various design changes and the like can be made without departing from the scope of this invention.

[Reference Signs List]

10, 10a Feature value calculation device
110 Signal information acquisition unit
111 Region setting unit
112 Feature value calculation unit
LS1 Scattered signal light
D1 Signal information
W1 Time waveform data
W2 Spectral waveform data
C1 Object to be measured

**Claims**

1. A feature value calculation device (10) that calculates a feature value indicating optical characteristics of an object to be measured (C1) in a flow cytometry method, the device (10) comprising:

   a signal information acquisition unit (110) to acquire signal information (D1) indicating a temporal change in intensity of the electromagnetic waves which is generated by a light detector receiving the modulated electromagnetic waves from the object to be measured (C1) which is present in a light irradiation region irradiated with the illumination light from the light source, by using any one or more of a configuration for structured illumination in which illumination light from a light source is structured as structured illumination light and radiated onto the object to be measured (C1) or a configuration for structured detection in which modulated electromagnetic waves from the object to be measured (C1) are structured and detected;

a region setting unit (111) that sets a region to be processed in waveform data in which a feature value is calculated in a domain of the waveform data based on the signal information (D1) acquired by the signal information acquisition unit (110); and

a feature value calculation unit (112) that calculates the feature value from the waveform data in the region set by the region setting unit (111).

2. The feature value calculation device (10) according to claim 1, wherein the waveform data is data indicating the intensity of the electromagnetic waves with elapsed time as a domain, and

the region setting unit (111) sets a region in a range of a specific elapsed time as the region in the waveform data.

3. The feature value calculation device (10) according to claim 1, wherein the waveform data is data indicating a signal intensity in a frequency spectrum with frequency as a domain, and

the region setting unit (111) sets a region in a range of a specific frequency as the region in the waveform data.

4. The feature value calculation device (10) according to claim 2, wherein measurement for performing discrimination based on morphological information of the object to be measured (C1) from the waveform data and measurement for calculating the feature value are executed in parallel on the basis of a discrimination model created in advance by learning a relationship between the waveform data and the morphological information of the object to be measured (C1) on the basis of machine learning.

5. The feature value calculation device (10) according to claim 1, wherein the signal information acquisition unit (110) acquires, as the signal information (D1), signal information indicating a temporal change in intensity of scattered light from the object to be measured (C1) which is present in the light irradiation region.

6. The feature value calculation device (10) according to claim 2, wherein the signal information acquisition unit (110) acquires, as the signal information (D1), signal information indicating a temporal change in intensity of scattered light from the object to be measured (C1) which is present in the light irradiation region,

the waveform data is data indicating the intensity of the scattered light with elapsed time as a domain,

the region setting unit (111) sets a region in a range of a specific elapsed time as the region in the waveform data, and

the feature value calculation unit (112) calculates, as the feature value, an integrated value of the intensity of the scattered light received by the light detector in the range of the specific elapsed time from the waveform data in the region set by the region setting unit (111).

7. The feature value calculation device (10) according to claim 1 or 2, further comprising a conversion unit that converts the feature value into a value corresponding to intensity of electromagnetic waves acquired by a flow cytometer other than its own device on the basis of the feature value calculated by the feature value calculation unit (112) and a measured value obtained by measurement of a reference object to be measured.

8. A method of calculating a feature value indicating optical characteristics of an object to be measured (C1) in a flow cytometry method, the method comprising:

a signal information acquisition step (S 10) of acquiring signal information (D1) indicating a temporal change in intensity of the electromagnetic waves which is generated by a light detector receiving the modulated electromagnetic waves from the object to be measured (C1) which is present in a light irradiation region irradiated with the illumination light from the light source, by using any one or more of a configuration for structured illumination in which illumination light from a light source is structured as structured illumination light and radiated onto the object to be measured (C1) or a configuration for structured detection in which modulated electromagnetic waves from the object to be measured (C1) are structured and detected;

a region setting step (S20) of setting a region to be processed in waveform data in which the feature value is calculated in a domain of the waveform data based on the signal information (D1) acquired in the signal information acquisition step (S10); and

a feature value calculation step (S30) of calculating the feature value from the waveform data in the region set within the region setting step (S20).

9. A program for causing a computer of a feature value calculation device (10) that calculates a feature value indicating optical characteristics of an object to be measured (C1) in a flow cytometry method to execute:

a signal information acquisition step (S 10) of acquiring signal information (D1) indicating a temporal change in intensity of the electromagnetic waves which is generated by a light detector receiving the modulated electromagnetic waves from the object to be measured (C1) which is present in a light irradiation region irradiated with the illumination light from the light source, by using any one or more of a configuration for structured illumination in which illumination light from a light source is structured as structured illumination light and radiated onto the object to be measured (C1) or a configuration for structured detection in which modulated electromagnetic waves from the object to be measured (C1) are structured and detected;

a region setting step (S20) of setting a region to be processed in waveform data in which the feature value is calculated in a domain of the waveform data based on the signal information (D1) acquired in the signal information acquisition step (S10); and

a feature value calculation step (S30) of calculating the feature value from the waveform data in the region set in the region setting step (S20).

FIG. 1

# FIG. 2A

# FIG. 2B

# FIG. 3

# FIG. 4

## FIG. 5

## FIG. 6

FIG. 7

# FIG. 8

FIG. 9

10a

FEATURE VALUE CALCULATION DEVICE

11a

CONTROL UNIT

D1

110
SIGNAL INFORMATION
ACQUISITION UNIT

111
REGION SETTING UNIT

112
FEATURE VALUE
CALCULATION UNIT

113
OUTPUT UNIT

114
CONVERSION UNIT

12
STORAGE UNIT

P1
MEASURED VALUE
INFORMATION

# FIG. 10

```
        ┌──────────────┐
        │    START     │
        └──────┬───────┘
               │
               ▼
   ┌──────────────────────────┐
   │ ACQUIRE SIGNAL INFORMATION│────S110
   └────────────┬─────────────┘
                │
                ▼
   ┌──────────────────────────┐
   │  SET REGION TO BE PROCESSED│────S120
   └────────────┬─────────────┘
                │
                ▼
   ┌──────────────────────────┐
   │  CALCULATE FEATURE VALUE  │────S130
   └────────────┬─────────────┘
                │
                ▼
   ┌──────────────────────────┐
   │   CONVERT FEATURE VALUE   │────S140
   └────────────┬─────────────┘
                │
                ▼
   ┌──────────────────────────┐
   │   OUTPUT FEATURE VALUE    │────S150
   └────────────┬─────────────┘
                │
                ▼
        ┌──────────────┐
        │     END      │
        └──────────────┘
```

FIG. 11

## FIG. 12

# EP 4 339 591 A1

## INTERNATIONAL SEARCH REPORT

| International application No. |
|---|
| **PCT/JP2022/017763** |

### A. CLASSIFICATION OF SUBJECT MATTER

*G01N 15/14*(2006.01)i; *G01N 21/49*(2006.01)i
FI:   G01N15/14 B; G01N21/49 A; G01N15/14 D

According to International Patent Classification (IPC) or to both national classification and IPC

### B. FIELDS SEARCHED

Minimum documentation searched (classification system followed by classification symbols)
   G01N15/14; G01N21/49

Documentation searched other than minimum documentation to the extent that such documents are included in the fields searched

   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2017/073737 A1 (UNIV TOKYO) 04 May 2017 (2017-05-04)<br>   paragraphs [0048], [0091], [0136]-[0141] | 1-6, 8-9 |
| A | | 7 |
| Y | WO 2018/203568 A1 (THINKCYTE INC) 08 November 2018 (2018-11-08)<br>   paragraphs [0064]-[0065], [0077]-[0100], fig. 8-9 | 1-6, 8-9 |
| A | | 7 |
| Y | WO 2006/103920 A1 (SYSMEX CORPORATION) 05 October 2006 (2006-10-05)<br>   paragraph [0034], claim 4 | 1-6, 8-9 |
| Y | US 2017/0322137 A1 (DEUTSCHES RHEUMA-FORSCHUNGSZENTRUM BERLIN) 09 November 2017 (2017-11-09)<br>   paragraphs [0081]-[0091] | 1-6, 8-9 |
| A | OTA, Sadao. Ghost cytometry. Science. 15 June 2018, 360, pp. 1246-51, DOI: 10.1126/science.aan0096<br>   entire text, all drawings | 1-9 |

☑ Further documents are listed in the continuation of Box C.        ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/017763**

**C.    DOCUMENTS CONSIDERED TO BE RELEVANT**

| Category* | Citation of document, with indication, where appropriate, of the relevant passages | Relevant to claim No. |
|---|---|---|
| A | WO 2016/136801 A1 (UNIV TOKYO) 01 September 2016 (2016-09-01)<br>entire text, all drawings | 1-9 |
| A | WO 2018/198586 A1 (SONY CORP) 01 November 2018 (2018-11-01)<br>entire text, all drawings | 1-9 |

Form PCT/ISA/210 (second sheet) (January 2015)

International application No.

**PCT/JP2022/017763**

| Patent document cited in search report | | | Publication date (day/month/year) | Patent family member(s) | | | Publication date (day/month/year) |
|---|---|---|---|---|---|---|---|
| WO | 2017/073737 | A1 | 04 May 2017 | US 2018/0327699 A1 paragraphs [0084], [0133], [0187]-[0195] | | | |
| | | | | EP | 3372985 | A1 | |
| | | | | CN | 108351289 | A | |
| WO | 2018/203568 | A1 | 08 November 2018 | US 2020/0150022 A1 paragraphs [0076]-[0077], [0089]-[0112], fig. 8-9 | | | |
| WO | 2006/103920 | A1 | 05 October 2006 | US 2008/0108103 A1 paragraphs [0093]-[0095], claim 4 | | | |
| | | | | EP | 1865303 | A1 | |
| | | | | CN | 101151517 | A | |
| US | 2017/0322137 | A1 | 09 November 2017 | EP | 3244191 | A1 | |
| WO | 2016/136801 | A1 | 01 September 2016 | US 2018/0246030 A1 entire text, all drawings | | | |
| | | | | US | 2021/0003498 | A1 | |
| | | | | EP | 3264031 | A1 | |
| | | | | CA | 2977481 | A1 | |
| | | | | CN | 107250716 | A | |
| | | | | CN | 111855621 | A | |
| WO | 2018/198586 | A1 | 01 November 2018 | US 2020/0124520 A1 entire text, all drawings | | | |
| | | | | EP | 3617691 | A1 | |
| | | | | CN | 110691964 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021080332 A
- WO 2016136801 A

- WO 2017073737 A